# EUROPEAN PATENT APPLICATION

(11) **EP 4 282 900 A1**
(43) Date of publication of application: **29.11.2023**
(21) Application number: 22305766.2
(22) Date of filing: 23.05.2022
(51) Int. Cl.: C08G 69/04, A61K 31/00, B82Y 5/00, C08G 69/10, C08L 77/04

(54) **AMPHIPHILIC POLY(AMINO ACID) BLOCK COPOLYMERS AND NANOPARTICLES THEREOF FOR DRUG DELIVERY APPLICATIONS**

(71) Applicant: Nanothera Biosciences, Inc., Mount Laurel, NJ 08054 (US)
(72) Inventor: LEBLEU, Coralie, 91650 BREUILLET (FR); PLET, Laetitia, 77123 NOISY-SUR-ECOLE (FR); PHILIPPE, Christiane, 91590 D'HUISON LONGUEVILLE (FR); ERRASTI, Gauthier, 91400 ORSAY (FR); DELACROIX, Thomas, 91540 MENNECY (FR); LECOMMANDOUX, Sébastien, 33610 CANEJAN (FR); CHAKRABARTI, Raj, MOORESTOWN, 08057 (US)
(74) Representative: Regimbeau

(57) **Abstract**

The present invention relates to the field of polymer chemistry and more particularly to poly(amino acid) copolymers and uses thereof for drug delivery applications. In particular, the present invention concerns a copolymer comprising a polysarcosine block, pSar, containing from 15 to 99 sarcosine constitutional units and a poly(amino acid) block, pAA, containing from 8 to 120 amino acid constitutional units.

## Description

### FIELD OF THE INVENTION

The present invention relates to the field of polymer chemistry and more particularly to poly(amino acid) copolymers and uses thereof for drug delivery applications.

### Background of the invention

Active pharmaceutical ingredients (APIs) often need to be mixed with one or several excipients to form ready to use drug products. Excipients are inactive ingredients that usually have a specific function in the drug form: they can be used for example as binders, lubricants, coating agents, or filling agents. Depending on the therapeutic indication, drugs can be administered through different routes (e.g. topical, oral, parenteral) which require different final drug dosage forms and thus the use of suitable excipients.

Polymer excipients are widely used in the formulation of drug products intended for every possible route of administration. They include natural compounds such as cellulose, semi-synthetic compounds such as cellulose derivatives, and synthetic polymers like poly(ethylene glycol) (PEG), polylactides, or polyamides. Polymer excipients can be used to modify the physical or chemical properties of an API, for example change its viscosity or increase its solubility.

Amphiphilic block copolymers are particularly interesting excipients to solubilize hydrophobic APIs in water. Constituted at least of one hydrophilic block covalently bound to one hydrophobic block, they self-assemble in water to form nano-sized aggregates at or above their critical aggregate concentration (CAC). These resulting supramolecular assemblies, also called nanoparticles (NPs), usually have a size ranging from 10 to 100 nm and can have various morphologies. For example, a micelle has a spherical core-shell structure where the hydrophobic blocks formed the hydrophobic core that is stabilized by a hydrophilic shell constituted of the hydrophilic blocks. A hydrophobic API can be loaded and segregated in the core during the micellization. API-loaded micelles prepared in diluents commonly used for parenteral administration such as saline can increase the solubility of the APIs without the need for co-solvents such as Cremophor^{®} EL (polyoxyethylated castor oil) that is known to cause several adverse effects. Another advantage is that the API is not covalently bound to the copolymer, this drug form could thus be applied to a broad range of APIs.

Furthermore, in addition to increasing the water-solubility of APIs, nanoparticles offer various advantages such as preventing the API premature degradation, controlling its release, improving its bioavailability, and for anti-tumor applications, enhancing its absorption into tumor tissues by passive targeting through the enhanced permeation and retention effect (EPR effect).

PEG is the most commonly used hydrophilic block due to its stealth effect. However, PEG is not biodegradable and can trigger immunogenic responses at high dosage or in long-term administration.

Polysarcosine (pSar) is a good alternative to PEG since they share similar properties such as hydrophilicity, stealthiness and low toxicity but pSar has the advantage of being biodegradable as it is based on the endogenous amino acid derivative sarcosine, N-methylated glycine.

Amphiphilic polysarcosine-poly(amino acid) copolymers have been recently developed for their biodegradability and ability to self-assemble in water into nanoparticles.

US10836869 discloses the use of such type of copolymers to solubilize hydrophobic molecules. These multiblock copolymers comprise a large number of sarcosine and amino acid units which lead to costly synthesis and post purification and industrial scale-up challenges.

Therefore, there is still a need for a polymer capable of solubilizing hydrophobic APIs that does not exhibit side effect when used for drug delivery applications.

### OBJECT OF THE INVENTION

One aspect of the invention is directed to a copolymer comprising a polysarcosine block, pSar, containing from 15 to 99 sarcosine constitutional units and a poly(amino acid) block, pAA, containing from 8 to 120 amino acid constitutional units.

A preferred embodiment is a copolymer having a hydrophilic fraction, f(pSar), ranging from 5 to 80%, preferably from 10 and 70%, wherein f(pSar) is the ratio in percentage of number average molar mass of the pSar block on the number average molar mass of the copolymer.

A preferred embodiment is a copolymer wherein amino acids of pAA block are D isomer and/or L isomer of amino acids constitutional units.

A preferred embodiment is a copolymer wherein amino acid constitutional units of pAA block is a hydrophobic amino acid preferably selected from the group comprising alanine, valine, norleucine, leucine, isoleucine, methionine, phenylalanine, tryptophan, proline, tyrosine, protected hydrophilic amino acids and combinations thereof.

A preferred embodiment is a copolymer chosen among formulas I and II, 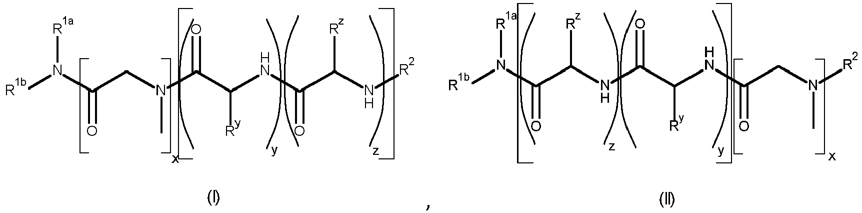 wherein
x is the number of sarcosine constitutional unit and is an integer ranging from 15 to 99, y+z is the number of amino acid constitutional units and is an integer ranging from 8 to 120,
groups R^{y} and R^{z} are independently chosen from an amino acid lateral-chain group, groups R^{1a} and R^{1b} are independently chosen from H, alkyl, alkenyl, alkynyl, cycloalkyl, aryl, heteroaryl,
group R² is chosen from H and a nitrogen protecting group.

A preferred embodiment is a copolymer, wherein
x is an integer ranging from 20 to 95, and preferably from 24 to 85,
y+z is an integer ranging from 9 to 110, preferably from 12 to 100.

A preferred embodiment is a copolymer, wherein
y+z is an integer ranging from 8 to 50, preferably from 9 to 45, preferably from 12 to 40, or
y+z is an integer ranging from 55 to 110, preferably from 60 to 100.

A preferred embodiment is a copolymer, wherein
y is a number ranging from 0 to 120, preferably 0 to 110, preferably from 5 to 100,
preferably from 9 to 95, preferably from 10 to 90, and
z is a number ranging from 0 to 120, preferably 0 to 110, preferably from 5 to 100,
preferably from 9 to 95, preferably from 10 to 90.

A preferred embodiment is a copolymer, wherein R^{y} and R^{z} are independently in D- or L-configuration, preferably R^{y} is the lateral chain of a hydrophobic L-amino acid, and R^{z} is the lateral chain of a hydrophobic D-amino acid, preferably R^{y} and R^{z} are independently chosen from the lateral chain of L-leucine, L-phenylalanine, L-tyrosine, γ-benzyl-L-glutamate, γ-tert-butyl-L-glutamate, L-cyclohexylglycine, D-leucine, D-phenylalanine, D-cyclohexylglycine, D-tyrosine, γ-benzyl-D-glutamate, γ-tert-butyl-D-glutamate.

Another aspect of the invention is directed to a method of preparation of a copolymer described above by polymerization of sarcosine derivates and amino acid derivates, wherein derivates are represented by formulas III and IV as follow 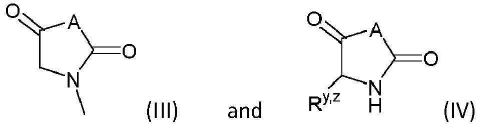 wherein
A is O or S,
R^{y}, R^{z} are as defined in any of claims 5 to 9.

Another aspect of the invention is directed to nanoparticles comprising copolymer as described above or prepared according to the method described above.

A preferred embodiment is nanoparticles further comprising at least one active compound, preferably selected from hydrophobic compounds, active pharmaceutical ingredients.

A preferred embodiment is nanoparticles, having
- a hydrodynamic diameter lower than 400 nm, preferably ranging from 5 to 200 nm, and/or
- a polydispersity index lower than 0.70, preferably ranging from 0.02 to 0.70; and/or
- a loading efficiency of more than 20%, preferably more than 30%.

Another aspect of the invention is directed to a method of preparing nanoparticles as described above comprising the following steps:
- preparing an organic solution containing the copolymer as described above,
- mixing the organic solution with an aqueous solution upon stirring,
- removing the organic solvent.

A preferred embodiment is a method of preparing nanoparticles wherein nanoparticles further comprise a hydrophobic compound, said method comprising the following steps:
- preparing an organic solution containing the copolymer as described above and at least a hydrophobic compound,
- mixing the organic solution with an aqueous solution upon stirring,
- removing the organic solvent.

### Definitions

The following are definitions of various terms used herein to describe the present disclosure and are further illustrated by the embodiments, sub-embodiments, and compounds disclosed herein. These definitions apply to the terms as they are used throughout this specification unless otherwise indicated in specific instances, either individually or as part of a larger group.

The term "halogen" or "halo", as used in the present invention, refers to a fluorine, chlorine, bromine or iodine atom.

The term "alkyl", as used in the present invention, refers to a straight or branched monovalent saturated hydrocarbon chain. "(C1-C6) alkyl" refers to alkyl containing from 1 to 6 carbon atoms.

The term "alkenyl" is given its ordinary meaning in the art and refers to an unsaturated hydrocarbon chain. "(C2-C6) alkenyl" refers to alkenyl containing from 2 to 6 carbon atoms that includes one or more carbon-carbon double bonds.

The term "alkynyl" is given its ordinary meaning in the art and refers to an unsaturated hydrocarbon chain. " (C2-C6) alkynyl" refers to alkynyl containing from 2 to 6 carbon atoms that includes one or more carbon-carbon triple bonds.

The term "cycloalkyl", as used in the present invention, refers to a hydrocarbon ring. "(C3-C10) cycloalkyl" refers to cycloalkyl having 3 to 10 carbon atoms including, but not limited to, cyclopropyl, cyclobutyl, cyclopentyl, cyclohexyl.

The term "aryl", as used in the present invention, refers to an aromatic hydrocarbon group preferably comprising 6 to 10 carbon atoms, comprising one or more fused rings and eventually one or more substituents such as halogen, (C1-C6) alkyl, -O(C1-C6) alkyl. For example, aryl includes phenyl, benzyl or naphthyl group.

The term "heteroaryl", as used in the present invention, refers to an aryl comprising one or several, notably one or two, preferably one, fused hydrocarbon cycles in which one or several, notably one to four, advantageously one or two, carbon atoms each have been replaced with a heteroatom selected from a sulfur atom, an oxygen atom and a nitrogen atom, preferably selected from an oxygen atom and a nitrogen atom, in particular a nitrogen atom. It can be a benzothiazolyl, furyl, thienyl, pyrrolyl, oxazolyl, isoxazolyl, thiazolyl, isothiazolyl, imidazolyl, pyrazolyl, oxadiazolyl, thiadiazolyl, triazolyl, tetrazolyl, pyridyl, pyridazinyl, pyrimidinyl, pyrazinyl, triazinyl, quinolyl, isoquinolyl, quinoxalyl or indolyl.

The terms "units" or "constitutional units" in the present disclosure refers to the constitutional units derived from polymerization of the monomer.

The terms "sarcosine constitutional unit" or "sarcosine unit" refer to unit derived from the monomer of sarcosine.

The terms "amino acid constitutional unit" or "amino acid unit" refer to unit derived from the monomer of amino acid.

The term "AA" is used to designate an amino acid. In the present disclosure, the term "AA" does not encompass sarcosine.

Amino acids are represented by formula NH₂-CHR-CO₂H, wherein R represents the lateral chain group of each amino acid. Amino acids used in the present invention can be natural amino acids or unnatural amino acids. The term "natural amino acids" refers to any naturally occurring amino acid that can be found in proteins or in nature. These are L-alanine, L-arginine, L-asparagine, L-aspartate, L-cysteine, L-glutamate, L-glutamine, glycine, L-histidine, L-isoleucine, L-leucine, L-lysine, L-methionine, L-phenylalanine, L-proline, L-pyrrolysine, L-selenocysteine, L-serine, L-threonine, L-tryptophan, L-tyrosine, and L-valine.

The term "unnatural amino acid" refers to any amino acid that is not included in the list of naturally occurring amino acids. Unnatural amino acid includes amino acid wherein the lateral chain "R" is chemically modified for example by functionalization or protecting groups.

The term "amino acid lateral chain group" also defined as "lateral chain group" refers to the group R on the α-carbon of any natural or unnatural AA, as defined herein. For example, the lateral chain group of L-alanine is a methyl, and the lateral chain group of γ-benzyl-glutamate is group - (CH₂)₂CO₂CH₂C₆H₅.

The term "D-amino acid" refers to an amino acid wherein the alpha carbon of the AA is in the D-configuration, also designated as D isomer, and the term "L-amino acid" refers to an amino acid wherein the alpha carbon of the AA is in the L-configuration, also designated as L isomer.

The term "poly(amino acid)" or "pAA" is used to designate a block made of a linear polymeric chain where the constitutional units are derived from amino acids or a mix of amino acids and are covalently linked by peptide bonds. A pAA can be formed via a ring-opening polymerization of monomers of amino acid. When only L-amino acid monomers are used, the resulting block is a poly(L-amino acid) block. When only D-amino acid monomers are used, the resulting block is a poly(D-amino acid) block. When a mix of L and D-amino acid is used in an unspecified sequence arrangement, the resulting block is a poly(L-amino acid-co-D-amino acid) block.

The term "Sar" is used to designate sarcosine.

The term "polysarcosine" or "pSar" is used to designate a block made of a linear polymeric chain where the constitutional units derive from sarcosine and are covalently linked by peptide bonds.

The term "monomer of amino acid" or "amino acid monomer" refers to an amino acid or any amino acid derivate which can be used in a polymerization process.

The term "monomer of sarcosine" refers to sarcosine or any sarcosine derivate which can be used in a polymerization process.

The term "sarcosine derivate" or "amino acid derivate" refers to any reactive form of sarcosine or amino acid which can be used in a polymerization process. For example, for a ring-opening polymerization, derivates can be a N-carboxyanhydride (NCA) of amino-acid or of sarcosine or a N-thiocarboxyanhydride (NTA) of amino-acid or of sarcosine.

The term "initiator" or "polymerization initiator" in the present disclosure refers to a molecule that reacts with a monomer to form a compound capable of reacting successively with other monomers by chain propagation to form a polymer.

The term "terminating agent" refers to a compound that reacts with the end of a living polymer chain and stops the chain propagation.

The term "capping agent" refers to a compound that reacts with the N-terminal part of the polymer chain to covalently bind a functional group such as -C(O)CH₃.

The terms "drug-loaded" and "loaded" refers to a particle comprising an API entrapped within the particle for example by encapsulation.

The expression "pharmaceutically acceptable" as used in the present invention is intended to mean what is useful to the preparation of a pharmaceutical composition, and what is generally safe and non-toxic, for a pharmaceutical use.

The terms "drug", "Active Pharmaceutical Ingredient", "API", "pharmaceutical" and derivatives thereof, are used interchangeably and refer to a substance intended for use in the diagnosis, cure, mitigation, treatment, or prevention of a disease.

The terms "particle" and "nanoparticle" according to the present invention are used interchangeably to designate an object obtained by the spontaneous self-assembly of block copolymers. For example, these nanoparticles can be micelles, polymersomes or polyplexes.

By "polymeric micelle" it is meant an object characterized by a core-shell structure with a hydrophobic core and a hydrophilic shell, formed by self-assembly of amphiphilic copolymers.

By "polymersome" it is meant a polymer-based vesicle which is an object with a structure of a bilayer enclosing an aqueous compartment, formed by self-assembly of amphiphilic copolymers.

By "polyplex" it is meant a polymeric system containing a complexed nucleic acid (eg DNA or RNA), formed by electrostatic interactions between cationic groups of a polymer and negatively charged nucleic acid.

It is understood that any one of the embodiments described below can be combined with any one of the other listed embodiments.

### METHODS

NCA polymerization can be monitored by Fourier-transform infrared spectroscopy (FTIR). For example, polymerizations are considered completed when NCA-associated carbonyl bands at 1850 and 1778 cm⁻¹ disappeared which correspond to the total consumption of NCAs.

The number of constitutional units of each monomer of pSar block and of pAA block can be determined by proton (¹H) nuclear magnetic resonance (NMR).

The copolymer's number average molar mass (Mₙ) and dispersity (D) can be determined by size-exclusion chromatography (SEC).

Hydrodynamic diameter (Dₕ) and polydispersity index (PDI) of the particles can be determined by dynamic light scattering (DLS).

The loading efficiency (LE) can be determined using ultra performance liquid chromatography (UPLC).

The loading content (LC) can be determined using SEC.

The water solubility of the API can be determined by any experimental or analytical method, such as UPLC.

Specific protocols for each method are given in the examples.

### DETAILED DESCRIPTION OF THE INVENTION

Surprisingly, it has been found that copolymers having a restricted number of Sar units are able to increase the solubility of active compounds and preferably of hydrophobic compounds such as hydrophobic APIs.

Advantageously, copolymers according to the invention are less expensive than copolymers of the prior art because their synthesis is facilitated. Furthermore, industrial scale-up of the copolymers according to the invention can also be facilitated.

In particular, copolymers according to the invention, via their ability to self-assemble into nanoparticles, are capable of improving the water solubility of hydrophobic compounds such as APIs preferably with a factor greater than 100, preferably greater than 200, preferably greater than 500, preferably greater than 1000, preferably greater than 2000 and more preferably greater than 3000.

### COPOLYMER

The invention relates to a copolymer comprising:
- a polysarcosine block, pSar, containing from 15 to 99 sarcosine constitutional units; and
- a poly(amino acid) block, pAA, containing from 8 to 120 amino acid constitutional units.

The person skilled in the art will understand that when the pAA block is formed by different AAs, the order of each AA unit can be statistical or controlled.

One advantage of copolymers according to the invention is that they are stable, biocompatible and easily degraded *in vivo.*

According to the invention, the pSar block of the copolymer is hydrophilic whereas the pAA block is less hydrophilic than the pSar block and thus has a hydrophobic behavior. Those characteristics render the copolymer amphiphilic and allow it to form stable nanoparticles such as micelles. Furthermore, nanoparticles formed by copolymers of the invention are biocompatible and easily degraded *in vivo.*

The amphiphilic character of the pSar-pAA copolymers according to the invention can be used to form nanoparticles. Advantageously, said nanoparticle can encapsulate an active compound for example to limit its degradation or to increase its solubility in water.

The limited number of constitutional units of the pSar-pAA copolymers of the invention makes the preparation of each block easy and cost-attractive.

pSar block contains a chain of from 15 to 99 sarcosine units, preferably from 20 to 95, preferably from 24 to 85 and more preferably from 28 to 80.

In one preferred embodiment, pSar block contains a chain of from 50 to 99 sarcosine units, preferably from 55 to 95, preferably from 60 to 85 and more preferably from 65 to 80.

In another preferred embodiment, pSar block contains a chain of from 15 to 50 sarcosine units, preferably from 18 to 45, preferably from 22 to 40 and more preferably from 25 to 35.

The AA of which the pAA are composed are preferably selected in the lists disclosed further below. The selection is made to ensure that the pAA block obtained by polymerization is less hydrophilic than the pSar block. The pAA block can be qualified as a hydrophobic block.

Preferably, the pAA block contains a chain from 8 to 120 AA units, preferably from 9 to 110, preferably from 12 to 100, and more preferably from 15 to 95.

In one preferred embodiment, the pAA block contains a chain from 8 to 50 AA units, preferably from 9 to 45, preferably from 12 to 40 and more preferably from 15 to 35.

In another preferred embodiment, the pAA block contains a chain from 50 to 120 AA units, preferably from 55 to 110, preferably from 60 to 100 and more preferably from 65 to 95.

Advantageously, the copolymer comprises two or more blocks wherein at least one block is pSar and at least one block is pAA. Preferably, the copolymer comprises two blocks wherein one block is pSar and one block is pAA.

Advantageously, the copolymer according to the invention has a hydrophilic fraction f(pSar) ranging from 5 to 80%, preferably from 10 to 70%, preferably from 15 to 60% and more preferably from 20 to 50%. The hydrophilic fraction, f(pSar), is the ratio in percentage of the number average molar mass (Mₙ) of the pSar block on the Mₙ of the copolymer.

According to one particular embodiment, the copolymer has a hydrophobic fraction f(pAA) ranging from 20 to 90%, preferably from 30 to 88%, preferably from 40 to 85% and more preferably from 50 to 80%. The hydrophobic fraction is the percentage of the Mₙ of the hydrophobic pAA block on the Mₙ of the copolymer.

Advantageously, the copolymer according to the invention has a number average molar mass, Mₙ, ranging from 500 g/mol to 50 000 g/mol, preferably from 800 to 45 000 g/mol, preferably from 1 000 to 40 000 g/mol, preferably from 1 200 to 35 000 g/mol and more preferably from 1400 to 32 000 g/mol.

Advantageously, the copolymer according to the invention has a dispersity, D, ranging from 1 to 2, preferably from 1.0 to 1.8 and more preferably from 1.0 to 1.6.

The hydrophobic pAA block comprises, preferably consists of, natural or unnatural amino acids units, AA units, as long as AA units are hydrophobic compared to the Sar unit of the pSar block. According to the invention, the pAA block does not comprise sarcosine units.

According to a preferred embodiment, amino acids of pAA block are D- or/and L- wherein D- refers to D isomer and L- refers to L isomer of amino acids. Preferably, amino acids are a mixture of D- and L- or only L- amino acids, or only D- amino acids. Preferably, when amino acids are a D-/L- mixture, the molar ratio between D-/L- is ranging from 30/70 to 70/30, preferably from 40/60 to 60/40, preferably from 45/55 to 55/45 and more preferably 50/50.

According to a preferred embodiment, AA of pAA is a hydrophobic amino acid selected from the group comprising alanine, valine, norleucine, leucine, isoleucine, methionine, phenylalanine, tryptophan, proline, tyrosine, protected hydrophobic amino acids, protected hydrophilic amino acids and combinations thereof.

Functional groups on the lateral chains of AA can be protected by different protecting groups.

Functional groups such as hydroxyl groups, amine groups, aldehyde groups and carboxylic acids groups, of amino acids lateral chain might be protected by esters, carbonates, sulfonates allyl esters, ethers, silyl ethers, alkyl ethers, arylalkyl ethers, alkoxyalkyl ethers. The esters used to protect hydroxyl groups according to the invention can be formates, acetates, proprionates, butanoates, pentanoates, crotonates, benzoates, benzoyle formate, chloroacetate, trifluoroacetate, methoxyacetate, triphenylmethoxyacetate, p-chlorophenoxyacetate, 3-phenylproprionate, 4-oxopentanoate, 4,4-(ethylenedithio)pentanoate, pivaloate (trimethylacetate), crotonate, 4-methoxy-crotonate, benzoate, p-benzylbenzoate, 2,4,6-trimethylbenzoate.

Advantageously, when carbonates are used to protect the hydroxyl group, said carbonates are chosen among 9-fluorenylmethyl, ethyl, 2,2,2-trichloro-ethyl, 2-(trimethylsilyl)ethyl, 2-(phenylsulfonyl)ethyl, vinyl, allyl, and p-nitrobenzyl carbonate.

Advantageously, when silyl ethers are used to protect the hydroxyl group, said silyl ethers are chosen among trimethylsilyl, triethylsilyl, tert-butyldimethylsilyl, tert-butyldiphenylsilyl, triisopropylsilyl ether, and other trialkylsilyl ethers.

Advantageously, when alkyl, alkoxyalkyl and arylalkyl ethers are used to protect the hydroxyl group; alkyl ethers are chosen among methyl, benzyl, p-methoxybenzyl, 3,4-dimethoxybenzyl, trityl, tert-butyl, and allyl ether, or their derivatives; Alkoxyalkyl ethers which include acetals are chosen among methoxymethyl, methylthiomethyl, (2-methoxyethoxy)methyl, benzyloxymethyl, beta-(trimethylsilyl)ethoxymethyl, and tetrahydropyran-2-yl ether; Arylalkyl ethers are chosen among benzyl, p-methoxybenzyl (MPM), 3,4-dimethoxybenzyl, o-nitrobenzyl, p-nitrobenzyl, p-halobenzyl, 2,6-dichlorobenzyl, p-cyanobenzyl, 2- and 4-picolyl ethers.

Advantageously, amine group of AA lateral chain can be protected by arylalkylamines, carbamates, allyl amines, amides, and their derivatives, including tert-butyloxycarbonylamino (-NHBOC), ethyloxycarbonylamino, methyloxycarbonylamino, trichloroethyloxycarbonylamino, allyloxycarbonylamino (-NHAlloc), benzyloxocarbonylamino (-NHCbz), allylamino, benzylamino (-NHBn), fluorenylmethylcarbonyl (-NHFmoc), formamido, acetamido, chloroacetamido, dichloroacetamido, trichloroacetamido, phenylacetamido, trifluoroacetamido, benzamido, tert-butyldiphenylsilyl.

Advantageously, aldehyde groups of AA lateral chain can be protected with acyclic acetals, hydrazones, imines, and more specifically with dimethyl acetal, diethyl acetal, diiso-propyl acetal, dibenzyl acetal, bis(2-nitrobenzyl) acetal, 1,3-dioxanes, 1,3-dioxolanes, semicarbazones, and their derivatives.

Advantageously, carboxylic acids groups of AA lateral chain can be protected with optionally substituted C1-C6 aliphatic esters, optionally substituted aryl esters, silyl esters, activated esters, amides, hydrazides, such as and not limited to, ethyl, propyl, isopropyl, butyl, isobutyl, benzyl, and phenyl ester, wherein each group is optionally substituted. Additional protected carboxylic acids include oxazolines and ortho esters.

Those skilled in the art would know that the hydrophilicity of AA can be transformed from hydrophilic into hydrophobic using protecting groups on the functional groups on the lateral chains of AA.

Hydrophilic AA, which can be later protected to become hydrophobic includes cysteine, tyrosine, serine, threonine, aspartic acid, glutamic acid, asparagine, lysine, histidine, arginine, glycine and glutamine.

Protecting groups are preferably selected from alkyl, alkoxyalkyl and arylalkyl ethers such as trityl, tert-butyl, benzyl; esters such as trifluoroacetate; arylalkylamines, carbamates, allyl amines, amides, and their derivatives, including tert-butyloxycarbonylamino; acetates.

Hydrophobic AA, preferably, includes β-trityl-asparagine, β-benzyl-aspartate, S-benzyl-cysteine, cyclohexylglycine, γ-benzyl-glutamate, γ-tert-butyl-glutamate, ε-trifluoroacetyl-lysine, ε-Boc-lysine, ε-benzyl-lysine, β-benzyl-serine, O-acetyl-tyrosine, O-benzyl-tyrosine. A preferred protected hydrophilic AA is γ-benzyl-glutamate.

According to one preferred embodiment, the copolymer is chosen among formulas I and II, 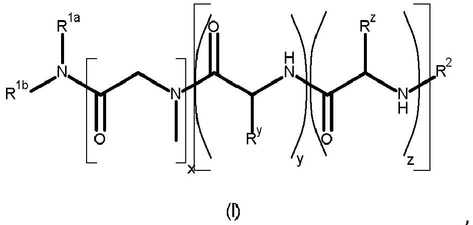 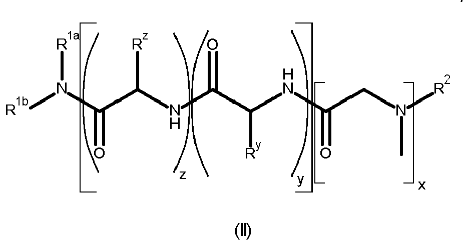 wherein
x is the number of sarcosine constitutional unit and is an integer ranging from 15 to 99,
y+z is the number of amino acid constitutional units and is an integer ranging from 8 to 120,
   groups R^{y} and R^{z} are independently chosen from an amino acid lateral-chain group,
   groups R^{1a} and R^{1b} are independently chosen from H, alkyl, alkenyl, alkynyl, cycloalkyl, aryl, heteroaryl,
   group R² is chosen from H and a nitrogen protecting group.
"x" is the total number of sarcosine constitutional units in pSar block. Preferably, x is an integer ranging from 20 to 95, preferably from 24 to 85 and more preferably from 28 to 80.

In one preferred embodiment, x is an integer ranging from 50 to 99 sarcosine units, preferably from 55 to 95, preferably from 60 to 85 and more preferably from 65 to 80.

In another preferred embodiment, x is an integer ranging from 15 to 50 sarcosine units, preferably from 18 to 45, preferably from 22 to 40 and more preferably from 25 to 35.

"y+z" is the total number of amino acid constitutional units. Preferably, y+z is an integer ranging from 9 to 110, preferably from 12 to 100 and more preferably from 15 to 95.

In one preferred embodiment, y+z is an integer ranging from 8 to 50, preferably from 9 to 45, preferably from 12 to 40 and more preferably from 15 to 35.

In one preferred embodiment, y+z is an integer ranging from 50 to 120, preferably from 55 to 110, preferably from 60 to 100 and more preferably from 65 to 95.

Advantageously, when x is ranging from 50 to 99, y+z is ranging from 50 to 120. Advantageously, when x is ranging from 55 to 95, y+z is ranging from 55 to 110. Advantageously, when x is ranging from 60 to 90, y+z is ranging from 60 to 100. Advantageously, when x is ranging from 65 to 85, y+z is ranging from 70 to 95.

Advantageously, when x is ranging from 15 to 50, y+z is ranging from 8 to 50. Advantageously, when x is ranging from 18 to 45, y+z is ranging from 9 to 45. Advantageously, when x is ranging from 22 to 40, y+z is ranging from 12 to 40. Advantageously, when x is ranging from 25 to 35, y+z is ranging from 15 to 35.

"y" is the number of AA units in pAA sub-block comprised in the pAA block. Preferably, "y" is a number ranging from 0 to 120, preferably 0 to 110, preferably from 5 to 100, preferably from 9 to 95, preferably from 10 to 90, preferably from 15 to 85, preferably from 18 to 80, preferably from 20 to 75, preferably from 25 to 70, and more preferably from 30 to 65.

"z" is the number of AA units in pAA sub-block comprised in the pAA block. Preferably, "z" is a number ranging from 0 to 120, preferably 0 to 110, preferably from 5 to 100, preferably from 9 to 95, preferably from 10 to 90, preferably from 15 to 85, preferably from 18 to 80, preferably from 20 to 75, preferably from 25 to 70, and more preferably from 30 to 65.

In some embodiments, y is a number ranging from 5 to 50 and z is a number ranging from 0 to 50. Preferably, y is ranging from 9 to 45 and z from 0 to 45. Preferably, y is ranging from 9 to 40 and z from 5 to 45. Preferably, y is ranging from 10 to 40 and z from 9 to 45. Preferably, y is ranging from 10 to 40 and z from 10 to 40.

In one preferred embodiment, y or z is 0. Preferably, z is 0 and y is ranging from 8 to 120, preferably from 9 to 110, preferably from 12 to 100, preferably from 15 to 95. In one preferred embodiment, z is 0 and y is an integer ranging from 8 to 50, preferably from 9 to 45, preferably from 12 to 40 and more preferably from 15 to 35. In one preferred embodiment, z is 0 and y is an integer ranging from 50 to 120, preferably from 55 to 110, preferably from 60 to 100 and more preferably from 65 to 95.

In formula (I) and (II), lateral chains of AA units of pAA block are represented by the group R^{y} and R^{z}. R^{y} and R^{z} groups are the lateral chains of AA units defined above. According to the invention, R^{y} and R^{z} are not H.

In one preferred embodiment, R^{y} and R^{z} groups of pAA block are independently chosen among natural or unnatural amino acids as long as the lateral chains of said hydrophobic pAA block are hydrophobic.

Alpha-carbons of the AA units which bear R^{y} and R^{z} groups can have a D- or L- configuration. In the present invention, when R^{y} or R^{z} is designated as L- or D-, preferably L-AA or D-AA, it means that the carbon bearing the group R^{y} or R^{z} is in L- or D- configuration. Preferably, when R^{y} or R^{z} is designated as L-AA or D-AA, it means that R^{y} or R^{z} is in L or D configuration of the corresponding AA.

According to one particular embodiment, alpha-carbons bearing R^{y} and R^{z} are independently in D-or L- configuration. Preferably, alpha-carbons bearing R^{y} and R^{z} have a different configuration. Preferably, R^{y} is the lateral chain of a hydrophobic L-AA, and R^{z} is the lateral chain of a hydrophobic D-AA. Preferably, R^{y} is the lateral chain of a hydrophobic D-AA, and R^{z} is the lateral chain of a hydrophobic L-AA.

Preferably, R^{y} and R^{z} are independently chosen from the lateral chain of L-leucine, L-phenylalanine, L-tyrosine, γ-benzyl-L-glutamate, γ-tert-butyl-L-glutamate, L-cyclohexylglycine, D-leucine, D-phenylalanine, D-cyclohexylglycine, D-tyrosine, γ-benzyl-D-glutamate, γ-tert-butyl-D-glutamate.

In some embodiments, each R^{y} is independently the lateral chain of L-leucine, L-phenylalanine, L-tyrosine, γ-benzyl-L-glutamate or γ-tert-butyl-L-glutamate. In some embodiments, each R^{z} is independently the lateral chain of D-leucine, D-phenylalanine, D-cyclohexylglycine, D-tyrosine, γ-benzyl-D-glutamate, γ-tert-butyl-D-glutamate. Preferably, each R^{y} is independently the lateral chain of γ-benzyl-L-glutamate or γ-tert-butyl-L-glutamate. In some embodiments, each R^{z} is independently the lateral chain of γ-benzyl-D-glutamate, γ-tert-butyl-D-glutamate.

In an embodiment, R² is a nitrogen protecting group. Nitrogen protecting group can be chosen from any group well-known in the state of the art. In some embodiments, nitrogen protecting groups include, but are not limited to arylalkylamines, carbamates, allyl amines, amides, and their derivatives, including tert-butyloxycarbonylamino (-NHBOC), ethyloxycarbonylamino, methyloxycarbonylamino, trichloroethyloxycarbonylamino, allyloxycarbonylamino (-NHAlloc), benzyloxocarbonylamino (-NHCbz), allylamino, benzylamino (-NHBn), fluorenylmethylcarbonyl (-NHFmoc), formamido, acetamido, chloroacetamido, dichloroacetamido, trichloroacetamido, phenylacetamido, trifluoroacetamido, benzamido, tert-butyldiphenylsilyl.

In a preferred embodiment of formula I or II, R^{1a} and R^{1b} are independently chosen from H, (C1-C10) alkyl, (C2-C10) alkene, (C2-C10) alkyne, (C3-C10) cycloalkyl, (C5-C10) aryl, (C4-C10) heteroaryl. Preferably, R^{1a} and R^{1b} are independently chosen from H, (C1-C6) alkyl, (C2-C8) alkene, (C2-C10) alkyne, (C3-C10) cycloalkyl, (C5 or C6) aryl, (C4 or C5) heteroaryl. More preferably, R^{1a} and R^{1b} are independently chosen from H, methyl, ethyl, n-propyl, iso-propyl, n-butyl, iso-butyl, sec-butyl, tert-butyl, n-pentyl, neopentyl, n-hexyl, cyclopropyl, cyclobutyl, cyclopentyl, cyclohexyl, and a group phenyl and/or benzyl optionally substituted with a halogen, - CH₃, -CF₃, -OCH₃, OH.

In a preferred embodiment of formula I or II, R^{1a} is H, R^{1b} is neopentyl and R² is selected from a H or -C(O)CH₃.

Advantageously, the group NR^{1a}R^{1b} is a residue derived from the polymerization initiator linked to a first block.

Advantageously, the group R² is a residue derived from a terminating agent or a capping agent linked to the last block formed during the copolymer's synthesis.

According to a preferred embodiment of the invention, when the first block is pSar and the last block is pAA, the copolymer is defined by general formula I, wherein
groups R^{1a} and R^{1b} are independently chosen from H, an alkyl group or an aryl group, group R² is chosen from H, alkyl group and a group selected from R³OC(O)-, R³C(O)-, R³SO₂-,
wherein R³ is selected from (C1-C10) alkyl, (C2-C10) alkene, (C2-C10) alkyne, (C3-C10) cycloalkyl, (C5-C10) aryl and (C4-C10) heteroaryl.

According to a preferred embodiment of the invention, when the first block is pAA and the last block is pSar, the copolymer is defined by general formula II, wherein
groups R^{1a} and R^{1b} are independently chosen from H, an alkyl group or an aryl group, group R² is chosen from H, alkyl group and a group selected from R³OC(O)-, R³C(O)-, R³SO₂-,
wherein R³ is selected from (C1-C10) alkyl, (C2-C10) alkene, (C2-C10) alkyne, (C3-C10) cycloalkyl, (C5-C10) aryl and (C4-C10) heteroaryl.

In a preferred embodiment, the group R² in formula I or II is a residue derived from a capping agent selected from an alkyl group and a group selected from R³OC(O)-, R³C(O)-, R³SO₂-. Preferably, R³ is selected from H, (C1-C6) alkyl, (C2-C8) alkene, (C2-C10) alkyne, (C3-C10) cycloalkyl, (C5 or C6) aryl, (C4 or C5) heteroaryl. More preferably R³ is selected from methyl, ethyl, propyl, butyl, trifluoromethyl.

In a preferred embodiment, copolymer is selected copolymer C1 to C12 of the examples represented by formulas (I') and (II') 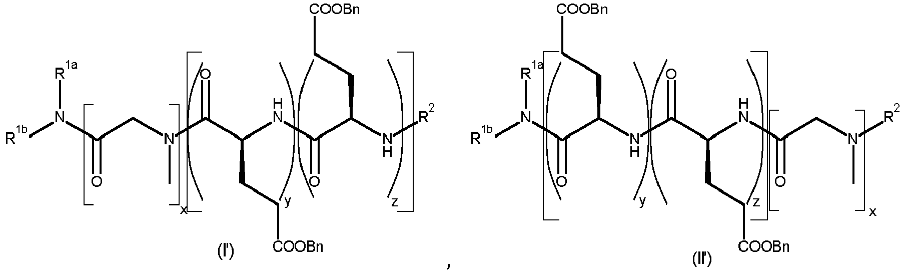 wherein
R² is H or CH₃C(O)-, and
x and "y+z" have different values depending on the copolymers according to the invention described in examples below.

### METHOD OF PREPARATION OF THE COPOLYMER

The present invention also relates to a method of preparation of copolymer. Preferably, copolymers are synthesized by polymerization of sarcosine derivates and amino acid derivates. Preferably, copolymers are synthesized by ring opening polymerization in which Sar and AA derivates are the respective N-carboxyanhydrides (NCAs) or thiocarboxyanhydrides (NTAs) of the corresponding constitutional units.

Advantageously, the synthesis of copolymers according to the invention having a limited number of constitutional units of Sar and/or AA is facilitated and thus less expensive compared to the prior art. Industrial scale-up of the preparation of the copolymers according to the invention is also facilitated.

According to the invention, Sar and AA derivates correspond to compounds represented respectively by formulas III and IV as follow 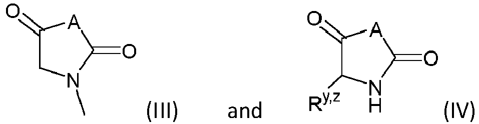 wherein A is O or S; and R^{y}, R^{z} are as described above.

Compounds of formula III and IV also called derivates, are NCAs when A is O and NTAs when A is S.

Compound of formula III can be the monomer used for preparing the pSar block. It is also designated as NCA/NTA of sarcosine or sarcosine NCA/NTA or Sar NCA/NTA.

Compound of formula IV can be the monomer used for preparing the pAA block. Compound IV is designated as IV^{y} when it is used to prepare units of pAA block having the superscript y. Similarly, compound IV^{z} is used to prepare units of pAA block having the superscript z. R^{y} and R^{z} are as described above. It is also designated as NCA/NTA of amino acid or amino acid NCA/NTA or AA NCA/NTA.

The copolymer of the present invention can be prepared by the method comprising
- a step a) of formation of a first block by ring opening polymerization by reacting a first derivate in the presence of a polymerization initiator, wherein the first derivate is chosen from compound III or at least one compound IV,
- an optional step b) of isolation of the first block formed in step a),
- a step c) of formation of a last block by mixing a second derivate chosen from compound III or at least one compound IV, with the first block obtained in step a) or isolated in the optional step b),
wherein the first and the second derivates are different and one of them is compound III,
- a step d) of recovery of the copolymer.

Advantageously, the step d) of recovery of the copolymer of the invention comprises the isolation and purification of the copolymer resulting from step c). Preferably, the step of recuperation d) is carried out after the consumption of the derivate introduced in step c).

In a preferred embodiment, when the last block is formed by addition of the derivate into the mixture obtained in step a), the step c) is carried out after the consumption of the derivate introduced in step a).

In a preferred embodiment, the derivate other than sarcosine introduced in step a) or c) is selected from two AAs having the formula IV. Preferably, one of amino acids acid has the formula IV^{y} with R^{y} group and the other AA has the formula IV^{z} with R^{z} group. R^{y} and R^{z} can be in a D- or L-configuration.

In a preferred embodiment, the copolymer can be prepared by one-pot method wherein the first block formed in step a) is not isolated in the optional step b). In this embodiment, the last block is formed by directly mixing the second derivate into the mixture obtained in step a) and after the consumption of the derivate introduced in step a).

In some embodiments, the derivate introduced in step a) is a compound III and the derivate introduced in step c) is a compound IV.

In another embodiment, the derivate introduced in step a) is a compound IV and the derivate introduced in step c) is a compound III.

Preferably, step a) and/or c) is/are carried out under reduced pressure or at atmospheric pressure.

Preferably, the step a) and/or c) is/are carried out at a temperature ranging from 0 to 80°C, preferably from 5 to 60°C, preferably from 10 to 50°C, preferably from 15 to 40°C, preferably from 20 to 30°C and more preferably from 22 to 28°C.

Advantageously, step a) and/or c) are carried in a solvent selected from N,N-dimethylformamide, tetrahydrofuran, dimethylsulfoxide, water, acetonitrile, ethyl acetate. Preferably, the solvent is N,N-dimethylformamide.

In some embodiments, the solvent of step a) is identical to the solvent of step c).

In a preferred embodiment, derivates are introduced in step a) and/or c) as solid powders.

In some embodiments, derivates are introduced in step a) and/or c) as solutions in a solvent. Preferably, the solvent of the derivates solution is identical to the solvent used for the reaction.

In some embodiments, the polymerization initiator is an amine preferably having the formula (R^{1a})(R^{1b})NH, wherein R^{1a} and R^{1b} are as described above.

In some embodiments, the molar ratio of the first derivate to polymerization initiator is ranging from 5 to 120, preferably from 20 to 110, preferably from 30 to 100 and more preferably from 40 to 95.

Advantageously, the method of preparation of the copolymer can also comprise a step of termination performed prior to step d) after the consumption of the derivate introduced in step c). Preferably, the step of termination corresponds to the consumption of all the derivate introduced in step c). Advantageously, the step of termination can be carried out with a terminating agent well known in the art. Preferably, the step of termination is performed by protonation. Then, R² is preferably H. Optionally, the terminating agent can be selected from water, an acid such as acetic acid, triflic acid or trifluoroacetic acid.

Preferably, the method of preparation comprises a step of capping carried out prior or after step d) by the addition of a capping agent and optionally any other reagents required for the reaction. When the capping step is prior to step d), the introduction of the capping agent is carried out after the consumption of the derivate introduced in step c). In this embodiment, the capping step can be carried out after the termination step. In this embodiment, capping agent can also be a terminating agent. Then, termination step and capping are performed in a single step.

When the capping step is after step d), said capping step is carried out by mixing the copolymer with the capping agent in presence of a solvent as defined above.

In any case, preferably, the capping step is carried out at a temperature ranging from 0 to 80°C, preferably from 5 to 60°C, preferably from 10 to 50°C and more preferably from 15 to 40°C, preferably from 20 to 30°C and more preferably from 22 to 28°C.

Advantageously, the capping step is performed at the same temperature than step a) and/or c). The capping step is preferably performed by addition of a capping agent having the formula R²X. R² is as described above, and X is a leaving group. In some embodiments, X is a leaving group well-known from the skilled person. Preferably, X is selected from CI-, I-, Br-, CF₃C(O)-, CH₃C(O)-.

### NANOPARTICLES

One other aspect of the invention is directed to nanoparticles comprising copolymers as defined above. Nanoparticles are formed by self-assembly of copolymers according to the invention. Advantageously, nanoparticles comprise copolymers and at least one active compound.

According to one aspect of the invention, nanoparticles according to the invention have a hydrodynamic diameter, Dₕ, lower than 900 nanometers (nm), preferably lower than 600 nm, preferably ranging from 1 to 600 nm, preferably from 2 to 500, preferably from 5 to 300 nm, preferably from 8 to 200 nm and more preferably from 10 to 110 nm.

According to one embodiment, the nanoparticles have a polydispersity index, PDI, lower than 0.7, preferably ranging from 0.02 to 0.70, preferably from 0.05 to 0.60 and more preferably from 0.10 to 0.50.

In a preferred embodiment, the active compound can be bound by non-covalent interactions with the nanoparticles. Preferably, the active compound can be bound within the nanoparticle or absorbed on its surface by non-covalent interactions. Preferably, the active compound is bound with the nanoparticle by hydrophobic interactions.

In a preferred embodiment, active compound is selected from hydrophobic compounds and/or active pharmaceutical ingredients (APIs). APIs include hydrophobic APIs and nucleic acids. According to one preferred embodiment, the nanoparticles comprise at least two active compounds, preferably at least two APIs.

In some embodiments, the active compound is a hydrophobic compound, preferably a hydrophobic API. Preferably, the hydrophobic compound is loaded within the nanoparticle.

Hydrophobic compound, preferably hydrophobic API, is a compound having a water-solubility of less than 10 g/L.

Preferably, the active compound is API. In one embodiment, the nanoparticles comprising copolymers and API are called drug-loaded nanoparticles.

Advantageously, drug-loaded nanoparticles according to the invention have the following advantages:
- prevent or delay the API degradation,
- controlling the API release,
- improving the API bioavailability, and
- for anti-tumor applications, enhancing the API absorption into tumor tissues by passive targeting through the enhanced permeation and retention (EPR effect).

Preferably, APIs are chosen to be suitable for targeting a disease selected by those skilled in the art. Examples of APIs include anticancer agents, antimicrobial agents, antiviral agents, antiinflammatory agents, immunosuppressive drugs, steroid drugs, hormone drugs, and antiangiogenic agents. These drug molecules may be used singly or in combination of two or more thereof.

In some embodiments, API is selected from 10,11-methylenedioxycamptothecin, 10-hydroxy-7-ethylcamptothecin (SN38), 2-chloroadenosine trimetrexate, 5-azacytosine, 5-azadeoxycytosine, 5'-deoxyfluorouridine, 5-fluorouracil (5-FU), 6-mercaptopurine, 6-thioguanine, 9-aminocamptothecin, 9-nitrocamptothecin, acyclovir, aldesleukin, allopurinol, amantadine, aminopterin, amsacrine, asparaginase, bleomycin, budesonide, busulfan, camptothecin, capecitabine, carboplatin, celecoxib, CI-973, cisplatin, cladribine, CPT-11, cyclophosphamide, cyclosporin, cytarabine, dacarbazine, daunorubicin, deoxycytidine, docetaxel, doxorubicin, eniluracil, epirubicin, epothilones A-E, etoposide, etoposide phosphate, etoposide phosphate, florafur, ganciclovir, gemcitabine (gemzar), ifosphamidemefosphamide, irinotecan, JM-216, karenitecin, karenitecin, lamivudine, L-phenylalanine mustard, methotrexate, methylene-10-deazaminopterin (MDAM), mitomycin, mitoxantrone, ormaplatin, oxaplatin, paclitaxel, perfosfamide, picoplatin, platinum-DACH, procarbazine, rapamycin, resveratrol, rimantadine, satraplatin, semustine, tamoxifen, TAS 103, temozolomide, tetraplatin, tomudex, topotecan, tretinoin, trophosphamide carmustine, UFT, valacyclovir, vinblastine, vincristine, vindesine, vinorelbine, zidovudine and combinations or a pharmaceutically acceptable salt thereof.

One aspect of the invention is the use of drug-loaded nanoparticles as a drug product, preferably in a cancer treatment. One preferred aspect of the invention is a method of treating or preventing a disease or a condition, such as cancer, comprising the administration to a patient in need thereof nanoparticles according to the invention. Another preferred aspect of the invention is the use of nanoparticles according to the invention for the manufacture of a drug product for a therapeutic application, preferably for the treatment of cancer. In particular, the hydrophobic API can be a cytotoxic agent, such as a taxoid and more particularly paclitaxel.

According to one particular embodiment, the hydrophobic API is advantageously an anticancer drug, preferably chosen among paclitaxel (taxol), doxorubicin, daunorubicin, vinblastine, docetaxel (taxotere), 10-hydroxy-7-ethylcamptothecin (SN38).

According to one aspect of the invention, nanoparticles improve the solubility of hydrophobic APIs. Water-solubility represents the concentration at which a compound is soluble in water. For a hydrophobic API loaded in nanoparticles, the water solubility of the API is considered to be the API concentration, [API], in the suspension of nanoparticles although it is not technically speaking in solution. Advantageously, nanoparticles improve the water solubility of hydrophobic APIs by a factor greater than 100, preferably greater than 200, preferably greater than 500, preferably greater than 1000, preferably greater than 2000 and more preferably greater than 3000.

In a preferred embodiment, the API in API-loaded nanoparticles has a water solubility, [API], from 0.2 to 2.0 g/L, preferably from 0.3 to 1.5 g/L, preferably from 0.4 to 1.5 g/L and more preferably from 0.5 to 1.5 g/L.

Advantageously, a high value of [API] permits to lower the volume of particle needed to be effective against the API's target such as a tumor.

### METHOD OF PREPARATION OF NANOPARTICLES

Copolymers according to the invention can spontaneously self-assemble in aqueous solutions into nanoparticles presenting a corona formed by the pSar block and a core formed by the pAA block.

One aspect of the invention concerns a method of preparation of nanoparticles comprising copolymers as described above. Preferably, the nanoparticles are obtained by a method comprising the following steps:
- preparing an organic solution containing the copolymer of the invention,
- mixing the organic solution with an aqueous solution,
- removing the organic solvent.

One preferred aspect of the invention is directed to a method of preparation nanoparticles comprising copolymer and an active compound as described above.

In the following, the method of preparation of nanoparticles is described for hydrophobic API but said method applies also for any hydrophobic compound as described above.

Advantageously, if the formation of nanoparticles is performed in presence of a hydrophobic API, it permits the encapsulation of said API in the hydrophobic core of the nanoparticle.

In some embodiments, the encapsulation of the hydrophobic API by said nanoparticles occurs with a method comprising the following steps:
- preparing an organic solution containing the copolymer as described above and at least one hydrophobic API as described above,
- mixing the organic solution with an aqueous solution,
- optionally removing the organic solvent,
- recovering the nanoparticles.

A preferred method of preparing nanoparticles comprises:
- a step i) of mixing a hydrophobic API and a copolymer according to the invention in a polar aprotic solvent in order to obtain an organic solution,
- a step ii) of mixing the solution obtained in step i) with an aqueous solution upon stirring in order to obtain nanoparticles,
- an optional step iii) of filtration of the nanoparticles,
- an optional step iv) of purification, for example by gel filtration chromatography,
- an optional step v) of filtration of the nanoparticles,
- an optional step vi) of freezing or freeze-drying,
- a step vii) of recovery of the nanoparticles.

Advantageously, nanoparticles according to the invention have a loading efficiency, LE, of more than 20%, preferably more than 30%, preferably more than 40%, preferably more than 45%, preferably more than 50%, preferably more than 55%, preferably more than 60%, preferably more than 65% and more preferably more than 70%. In some embodiments, the loading efficiency is between 40 and 99%, preferably between 50 and 96%, preferably between 60 and 95% and more preferably between 70 and 94%.

The loading efficiency is the weight ratio of the mass of API loaded into nanoparticles on the mass of feeding API.

Advantageously, the feed weight ratio, FWR, is ranging from 1 to 100%, preferably from 2 to 80%, preferably from 3 to 60%, preferably from 4 to 50%, preferably from 5 to 40% and more preferably from 10 to 30%. FWR is the weight ratio of the mass of feeding API on the mass of feeding copolymer.

In one embodiment, step ii) is carried out by adding the organic solution obtained in step i) into an aqueous solution.

In another embodiment, step ii) is carried out by adding the aqueous solution into the solution obtained in step i).

In some embodiments, the polar aprotic solvent is selected from N,N-dimethylformamide and N,N-dimethylacetamide.

In a preferred embodiment, the polar aprotic solvent used in step ii) is identical to the solvent used in step a) and/or c) of the preparation of the copolymer. This embodiment facilitates the purification of the copolymers and limits the potential effect of the residual solvent coming from the preparation of the copolymer on the self-assembly into nanoparticles. Preferably, the solvent is N,N-dimethylformamide.

In a preferred embodiment, wherein nanoparticles are suitable as a pharmaceutical dosage form, the aqueous solution of step ii) is selected from a solution comprising a cryo-protectant, a buffer and water. Preferably, the aqueous solution is water.

The cryo-protectant prevents the damage or alteration of other compounds related to freezing or freeze-drying and allows to reach a physiological osmolarity. Cryo-protectant includes, but is not limited to: monosaccharides, disaccharides, polyalcohols, amino acids, glycine, polyvinyl pyrrolidine, polyethylene glycol, mannitol, sorbitol, sucrose, glucose, raffinose, sucralose, lactose, trehalose, dextran, and dextrose. Preferably, cryo-protectant is trehalose.

The buffer allows to reach a physiological pH and also has an impact on osmolarity. The buffer includes, but is not limited to: a phosphate buffer, a phosphate buffer saline (PBS), a histidine buffer or HEPES buffer.

Advantageously, the mixing conditions in step ii) are modulated to optimize the resulting nanoparticles. Said conditions are for example the nature of mixing (magnetic stirring, homogenizer), the mixing time, the order of addition of one solution to the other or the flow rate of addition.

The optional step iii) can be performed to remove unloaded (free) API from the suspension in order to start the purification process and preserve the life time of the column used in optional step iv).

The optional step iv) can be performed to obtain a nanoparticle suspension in pure aqueous solution by removing the polar protic solvent and unloaded API.

In a preferred embodiment, a filtration at step v) is carried out with a 0.2 µm, 0.22 µm or 0.45 µm sterile filter in order to sterilize the nanoparticles.

### EXAMPLES

In order that the disclosure described herein may be better understood, the following examples are provided. It is understood that these examples are for illustrative purposes only and should not be construed as limiting this disclosure in any manner.

In the following examples copolymers according to the invention comprise a block of poly(sarcosine) and a block derived from γ-benzyl-L-glutamate and/or γ-benzyl-D-glutamate.

### Materials and Methods

Paclitaxel was supplied by Key Organics. All the organic solvents (HPLC grade) were provided by VWR. Water was ultrapure grade. Unless stated otherwise, the temperature was room temperature (22 - 28°C).

### FTIR SPECTROSCOPY

NCA polymerization was monitored by Fourrier-transform infrared (FTIR) spectroscopy on a Thermo Scientific Nicolet iS5 spectrometer equipped with an ID7 ATR module. Data were processed using OMNIC 9.7 software. Polymerizations were stopped when NCA-associated carbonyl bands at 1850 and 1778 cm⁻¹ had disappeared, corresponding to the total NCA consumption.

### PROTON NMR

Proton Nuclear Magnetic Resonance (¹H NMR) was performed on a 80 MHz MAGRITEK Spinsolve 80 Carbon, with the following parameters: NS = 64, repetition time = 10 to 30 s, pulse angle = 90, by dissolving the product in deuterated dimethylsulfoxide (d₆-DMSO) at a concentration between 20 and 50 g/L. Data were processed using SpinSolve and MestReNova softwares.

### SEC

Size-exclusion chromatography (SEC) analyses were performed in DMF containing 0.45 %w/v LiBr on a Agilent 1260 LC equipped with a diode array detector (UV-Vis) and a differential refractive index detector (dRI) and a three-column set of PSS GRAM analytical columns (100 Å, 8 x 300 mm, 10 µm; 100 Å, 8 x 300 mm, 10 µm; 1000 Å, 8 x 300 mm, 10 µm) with exclusion limits from 100 to 1 000 000 g/mol. Analyses were carried out on samples prepared in DMF containing 0.45 %w/v at 45°C using DMF containing 0.45 %w/v LiBr as eluent (1 mL/min). Data was acquired and processed with OpenLAB Chemstation software.

### SEC for the characterization of copolymers

To determine the number average molar mass (Mₙ) and dispersity (D) of polymers and copolymers, analyses were carried out on polymer samples prepared at 4 g/L. EasiVial kit of polystyrenes from Agilent was used as standard (266 to 66 000 g/mol). And data was further processed with Cirrus add-on.

### SEC for the characterization of nanoparticles

To determine the copolymer concentration in the PTX-loaded nanoparticles ([C in NPs]), a volume of 400 µL of each sample of NPs was dried using a centrifugal evaporator (SP Genevac EZ2, water evaporation automatic program). The samples injected in SEC were prepared by dissolving the dried sample in 1 mL of DMF containing 0.45 %w/v LiBr, hence a dilution by 2.5.

A standard curve in DMF containing 0.45 %w/v LiBr was prepared for each copolymer with polymer concentrations ([C]) between 1 and 4 g/L. Linear regression of the area obtained for the RI signal of the polymer versus [C] was plotted for each polymer to obtain the standard curve equation.

For each formulated sample, [C in NPs] was determined using the peak area obtained and the appropriate standard curve equation, and then multiplying by the factor of dilution.

Loading content (LC) of the PTX-loaded NPs was calculated as the ratio of the mass of PTX recovered at the final stage of the NPs' preparation (m(PTX in NPs), obtained by UPLC) divided by the mass of the NPs; the mass of NPs being the sum of the mass of copolymer (obtained using [C in NPs] by SEC) and m(PTX in NPs).

### DLS

DLS measurements were carried out on a Zetasizer Pro (Malvern Panalytical) equipped with a He-Ne laser (633 nm), at 25 °C and a scattering angle of 174.8°. Software used was ZS Explorer. A low volume plastic cell of 10 mm optical path length was filled with 70 µL of sample. Viscosity of the dispersant was corrected according to the solvent or mixture of solvents used. Data was acquired on three different measurements with an automatic optimization of the number and duration of runs per measurement. Results are expressed as an average of these 3 measurements. Dₕ of the objects is the intensity mean for each population. PDI is calculated from the autocorrelation functions using the cumulant method.

### UPLC

Ultra-performance liquid chromatography (UPLC) measurements were performed on an Acquity UPLC H-class from Waters equipped with a reversed-phase column (Acquity BEH, C18, 130 Å, 50 mm x 2.1 mm x 1.7 µm, Waters) and a diode array detector (DAD) Acquity eλ from Waters. Data was acquired and processed with Empower 3 software. The gradient elution was performed with 2 solvents: solvent A was water containing 0.05 vol% trifluoroacetic acid (TFA) and solvent B acetonitrile (ACN) containing 0.05 vol% TFA. Column was equilibrated for at least 30 min at a A:B mixture of 80:20. The sequence duration was 8 min with the following gradient of A:B solvents: 0 - 3 min 80:20, 3 - 5 min 5:95, 5 - 8 min 80:20. Eluents were degassed by the machine. Flow rate was 0.5 mL/min. Column temperature was 35°C. Injected volume was 3 µL. UV detector was set at 254 nm. Typical retention time of PTX was 2.24 min.

Loading efficiency (LE) of the PTX-loaded NPs was determined by UPLC. The injected samples were prepared by dissolving 90 µL of NPs in 910 µL of ACN:DMF 90/10 v/v, hence a dilution by 11.1.

A standard curve in ACN:(H₂O/DMF 90/10) 90:10 v/v was prepared with PTX concentrations ([PTX]) between 5 and 20 µg/mL. Linear regression of the area at 254 nm versus [PTX] was plotted to obtain the standard curve equation.

For each formulated sample, PTX concentration in nanoparticles ([PTX in NPs]) was determined using the peak area obtained and the standard curve equation, and then multiplying by the factor of dilution.

LE was calculated as the ratio of m(PTX in NPs) (obtained using [PTX in NPs]) divided by the mass of PTX initially fed in the NPs' preparation.

### HS-GC

Headspace gas chromatography (HS-GC) was used to dose the content of DMF as a residual solvent in formulations. HS-GC were performed on an Agilent 7890B GC system equipped with split/splitless injector, a flame ionization detector (FID), an Agilent 7697A autosampler and using a CP Sil 5CB column (50 m length, 0.32 mm diameter, 5 µm film thickness). A known quantity of sample was introduced into a 20 mL crimp vial and dissolved with a few milliliters of a low volatile solvent (water, DMSO or NMP). The vial was crimped and incubated in order to reach an equilibrium of concentration of the residual solvent between the liquid phase and the gas phase. The headspace of the vial was then injected into the GC system via a split injector and the residual solvent was detected by FID. Data were acquired and processed using OpenLAB Chemstation software.

### ABBREVIATIONS

The following abbreviations have been used in the examples:
- [C]: Copolymer concentration
- [C in NPs]: Copolymer concentration in the nanoparticles
- [PTX]: PTX concentration
- [PTX in NPs]: PTX concentration in the nanoparticles
- ¹H NMR: Proton Nuclear Magnetic Resonance
- D: Dispersity
- D-GluOBzl: γ-benzyl-D-glutamate
- Dₕ: Hydrodynamic diameter
- DLS: Dynamic light scattering
- DMAP: Dimethylaminopyridine
- DMF: N,N-Dimethylformamide
- DMSO: Dimethylsulfoxide
- eq: Equivalent
- f(pSar): Hydrophilic (polysarcosine) fraction
- FTIR: Fourrier-transform infrared
- FWR: Feed weight ratio
- HS-GC: Headspace gas chromatography
- LC: Loading content
- LE: Loading efficiency
- L-GluOBzl: γ-benzyl-L-glutamate
- Mₙ: Number average molar mass
- MTBE: Methyl tert-butyl ether
- NCA: N-carboxyanhydride
- NMM: N-Methylmorpholine
- NP: Nanoparticle
- PDI: polydispersity index
- PES: Polyethersulfone
- pSar: Polysarcosine
- PTX: Paclitaxel
- Sar: Sarcosine
- SEC: Size exclusion chromatography
- TMS: Tetramethylsilane
- UPLC: Ultra-performance liquid chromatography

In the following examples, in accordance with methods described above,
- values of x, y+z for each copolymer and f(pSar) were determined by ¹H NMR;
- Mₙ and Ð were determined by SEC;
- Dₕ and PDI were determined by DLS;
- [PTX] and LE were determined by UPLC;
- LC was determined by SEC.

### EXAMPLE 1: PREPARATION OF PSAR BLOCKS

Sar NCA (4.34.10⁻² mol, 75 eq) was dissolved in dry DMF at room temperature followed by the addition of neopentylamine as initiator (73 µL, 6.20.10⁻⁴ mol, 1 eq). The reaction mixture was stirred at room temperature and after the end of CO₂ evolution, completion of the reaction was confirmed by FTIR spectroscopy.

Precipitation of the polymer was performed at room temperature by pouring the reaction mixture on 300 mL of ethyl acetate upon vigorous stirring. After filtration, the product was reslurried with 2 x 100 mL of ethyl acetate and then dried under vacuum.

pSar block S1 with x = 75 was obtained with a yield of 70%, Mₙ of 4570 g/mol and D of 1.16.

pSar block S2 was prepared following the same procedure except that 28 eq of Sar NCA (1.62.10⁻² mol) was used. pSar block S2 with x = 29 was obtained with a yield of 85%, Mₙ of 2200 g/mol and D of 1.22.

### EXAMPLE 2: PREPARATION OF COPOLYMERS OF FORMULA I

### Procedure for two-step synthesis

pSar block S1 synthetized in example 1 (Mₙ = 4570 g/mol, 1.31.10⁻⁴ mol, 1.0 eq) was dissolved in dry DMF (17 mL) at room temperature. A mixture of L-GluOBzl NCA (6.42.10⁻³ mol, 49 eq) and D-GluOBzl NCA (6.42.10⁻³ mol, 49 eq) powders was added to the reaction medium. The reaction mixture was stirred at 5 °C and after the end of CO₂ evolution, completion of the reaction was confirmed by FTIR spectroscopy.

Precipitation of the copolymer was performed at room temperature by pouring the reaction mixture on 200 mL of MTBE upon vigorous stirring. After filtration, the product was reslurried with 2 x 100 mL of MTBE and then dried under vacuum. Copolymer 1 was obtained with a yield of 76%.

Copolymers C2 to C5 according to the invention were prepared following the same procedure as C1 with the pSar block and quantities presented in Table 1.

**Table 1: preparation conditions of copolymers C2 to C5.**

| Copolymer | Yield (%) | pSar block | n of pSar block (mol) | D-GluOBzl NCA (eq) | L-GluOBzl NCA (eq) | DMF (mL) |
|---|---|---|---|---|---|---|
| C2 | 84 | S1 | 4.40.10⁻⁴ | 12 | 12 | 25 |
| C3 | 70 | S2 | 1.00.10⁻⁴ | 4.5 | 4.5 | 25 |
| C4 | 73 | S2 | 0.50.10⁻⁴ | 18 | 18 | 40 |
| C5 | 83 | S1 | 3.85.10⁻⁴ | 24 | 0 | 20 |

Copolymers C1 to C5 according to the invention have the characteristics presented in table 2.

**Table 2: characteristics of copolymers C1 to C5 according to the invention.**

| Copolymer | x | y+z | f(pSar) (%) | Mₙ (g/mol) | D |
|---|---|---|---|---|---|
| C1 | 58 | 79 | 19 | 23770 | 1.26 |
| C2 | 63 | 22 | 48 | 9223 | 1.17 |
| C3 | 28 | 9 | 50 | 4961 | 1.22 |
| C4 | 28 | 33 | 21 | 8695 | 1.45 |
| C5 | 66 | 23 | 48 | 9832 | 1.36 |

### Procedure for "one-pot" synthesis

Sar NCA (2.78.10⁻² mol, 70 eq) was dissolved in dry DMF at room temperature followed by the addition of neopentylamine as initiator (1 eq). The reaction was stirred at room temperature and after the end of CO₂ evolution, completion of the reaction was confirmed by FTIR spectroscopy. To the reaction medium containing pSar was added L-GluOBzl NCA (4.52.10⁻³ mol, 12 eq) and D-GluOBzl NCA (4.52.10⁻³ mol, 12 eq) previously weighted and mixed. After 20h at room temperature, total consumption of NCAs was confirmed by FTIR spectroscopy.

Precipitation of the copolymer was performed at room temperature by pouring the reaction mixture on 300 mL of MTBE upon vigorous stirring. After filtration, the product was reslurried with 2 x 100 mL of MTBE and then dried under vacuum. Copolymer 6 was obtained with a yield of 54%.

The characteristics of copolymer C6 are presented in table 3.

**Table 3: characteristics of copolymer C6 according to the invention.**

| Copolymer | x | y + z | f(pSar) (%) | Mₙ (g/mol) | D |
|---|---|---|---|---|---|
| C6 | 76 | 82 | 58 | 7893 | 1.21 |

### EXAMPLE 3: PREPARATION OF COPOLYMERS OF FORMULA II

### Procedure for "reverse one-pot" synthesis

L-GluOBzl NCA (11 eq) and D-GluOBzl (11 eq) were dissolved in 40 mL of dry DMF at room temperature followed by the addition of neopentylamine as initiator (58.5 µL, 1 eq). The reaction mixture was stirred at room temperature and after the end of CO₂ evolution, completion of the reaction was confirmed by FTIR spectroscopy.

Sar NCA (70 eq) was then added to the reaction mixture. Again, the reaction mixture has been stirred at room temperature and after the end of CO₂ evolution, completion of the reaction is confirmed by FTIR spectroscopy. Precipitation of the copolymer was performed at room temperature by pouring the reaction mixture on 200 mL of MTBE upon vigorous stirring. After filtration, the product was reslurried with 2 x 100 mL of MTBE and then dried under vacuum. Copolymer C7 was obtained with a yield of 89%.

Copolymer C8 was prepared following the same procedure as C7 using L-GluOBzl NCA (24 eq) and no D-GluOBzl NCA. Copolymer C8 was obtained with a yield of 91%.

Copolymer C9 was prepared following the same procedure as C7 using D-GluOBzl NCA (24 eq) and no L-GluOBzl NCA. Copolymer C9 was obtained with a yield of 85%.

The characteristics of copolymers C7 to C9 are presented in table 4.

**Table 4: characteristics of copolymers C7 to C9 according to the invention.**

| Copolymer | x | y + z | f(pSar) (%) | Mₙ (g/mol) | D |
|---|---|---|---|---|---|
| C7 | 75 | 25 | 50 | 6985 | 1.38 |
| C8 | 80 | 23 | 53 | 10134 | 1.22 |
| C9 | 70 | 23 | 50 | 9926 | 1.11 |

### Procedure of capping

Copolymer C8 was dissolved in 10 mL of dry DMF at room temperature followed by the addition of DMAP (16 mg, 1.3.10⁻⁴ mol, 1 eq), and NMM (162 µL, 1.43.10⁻³ mol, 11 eq). After full dissolution, acetic anhydride (123 µL, 1.3.10⁻³ mol, 10 eq) was added and the reaction mixture was stirred overnight at room temperature.

Precipitation of the copolymer C10 was performed at room temperature by pouring the reaction mixture on 50 mL of MTBE upon vigorous stirring. After filtration, the product was reslurried with 2 x 10 mL of MTBE and then dried under vacuum. Copolymer C10 was obtained with a yield of 83%.

Copolymer C11 was prepared following the same procedure as C10 using C7 as starting material. Copolymer C11 was obtained with a yield of 87%.

Copolymer C12 was prepared following the same procedure as C10 using C9 as starting material. Copolymer C12 was obtained with a yield of 81%.

The characteristics of copolymer C10 to C12 are presented in table 5.

**Table 5: characteristics of copolymer C10 to C12 according to the invention.**

| Copolymer | x | y + z | f(pSar) (%) | Mₙ (g/mol) | D |
|---|---|---|---|---|---|
| C10 | 80 | 23 | 53 | 11442 | 1.19 |
| C11 | 68 | 22 | 50 | 8242 | 1.23 |
| C12 | 68 | 22 | 50 | 10559 | 1.09 |

### EXAMPLE 4 : PREPARATION OF PACLITAXEL-LOADED NANOPARTICLES

Paclitaxel (PTX) loaded nanoparticles were formed by a solvent-displacement method, also called nanoprecipitation.

A solution 1 of copolymer C1 to C12 in DMF at a concentration of 200 g/L was prepared. A solution 2 of PTX in DMF at a concentration of 20 g/L was prepared. Using solutions 1 and 2, a solution 3 at a feed weight ratio (FWR) of 10% was prepared, containing [C] = 100 g/L and [PTX] = 10 g/L in DMF.

Using a syringe pump (Fusion 100-X, Chemyx), 1 mL of the solution 3 was injected at a flow rate of 30 mL/min in 9 mL of water under 400 rpm stirring. After complete addition, stirring was pursued for 5 min.

The freshly obtained suspensions were purified to remove DMF and unloaded drug by a first filtration on a 0.20 µm PES syringe filter, followed by a gel filtration column (PD-10 desalting, Cytiva) using water as eluent, and a final filtration on a 0.20 µm PES syringe filter. The resulting NP1 to NP12 nanoparticles have the characteristics detailed in table 6.

**Table 6: characteristics of PTX-loaded nanoparticles NP1 to NP12 according to the invention.**

| Nanoparticles | Copolymer | Dₕ (nm) | PDI | LE | [PTX in NPs] (g/L) | LC | DMF content (ppm) |
|---|---|---|---|---|---|---|---|
| NP1 | C1 | 83 | 0.26 | 75% | 0.59 | 11% | nd |
| NP2 | C2 | 22 | 0.30 | 67% | 0.52 | 8% | 34 |
| NP3 | C3 | 27 | 0.08 | 68% | 0.51 | 8% | 23 |
| NP4 | C4 | 109 | 0.24 | 68% | 0.43 | 7% | 23 |
| NP5 | C5 | 30 | 0.21 | 84% | 0.58 | 14% | 30 |
| NP6 | C6 | 47 | 0.24 | 64% | 0.46 | / | / |
| NP7 | C7 | 10 | 0.16 | 73% | 0.57 | 8% | 19 |
| NP8 | C8 | 16 | 0.25 | 76% | 0.55 | 12% | 28 |
| NP9 | C9 | 9 | 0.26 | 68% | 0.46 | 8% | 31 |
| NP10 | C10 | 23 | 0.03 | 73% | 0.57 | 11% | 19 |
| NP11 | C11 | 29 | 0.17 | 64% | 0.49 | 7% | nd |
| NP12 | C12 | 23 | 0.03 | 72% | 0.48 | 8% | 17 |

| | | | | | | | |
|---|---|---|---|---|---|---|---|
| /: not determined nd: not detected (below limit of detection) | | | | | | | |

PTX solubility in water is lower than 0.1 µg/mL. Results in table 6 show that nanoparticles according to the invention allow to significantly increase PTX water-solubility.

Advantageously, nanoparticles according to the invention present only traces of DMF, which is compatible with their use in pharmaceutical applications.

## Claims

1. A copolymer comprising a polysarcosine block, pSar, containing from 15 to 99 sarcosine constitutional units and a poly(amino acid) block, pAA, containing from 8 to 120 amino acid constitutional units.

2. A copolymer according to claim 1, having a hydrophilic fraction, f(pSar), ranging from 5 to 80%, preferably from 10 and 70%, wherein f(pSar) is the ratio in percentage of number average molar mass of the pSar block on the number average molar mass of the copolymer.

3. A copolymer according to any one of claim 1 or 2, wherein amino acids of pAA block are D isomer and/or L isomer of amino acids constitutional units.

4. A copolymer according to any one of claims 1 to 3, wherein amino acid constitutional units of pAA block is a hydrophobic amino acid preferably selected from the group comprising alanine, valine, norleucine, leucine, isoleucine, methionine, phenylalanine, tryptophan, proline, tyrosine, protected hydrophilic amino acids and combinations thereof.

5. A copolymer according to any one of claims 1 to 4, chosen among formulas I and II, 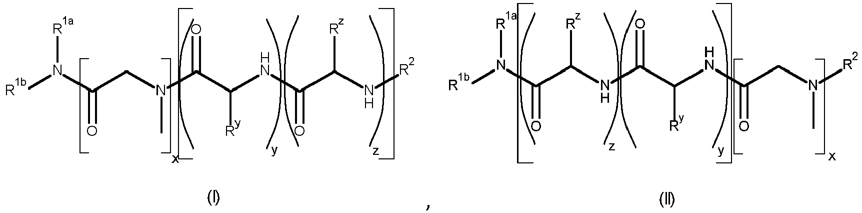 wherein
x is the number of sarcosine constitutional unit and is an integer ranging from 15 to 99,
y+z is the number of amino acid constitutional units and is an integer ranging from 8 to 120,
groups R^{y} and R^{z} are independently chosen from an amino acid lateral-chain group,
groups R^{1a} and R^{1b} are independently chosen from H, alkyl, alkenyl, alkynyl, cycloalkyl, aryl, heteroaryl,
group R² is chosen from H and a nitrogen protecting group.

6. A copolymer according to claim 5, wherein
x is an integer ranging from 20 to 95, and preferably from 24 to 85,
y+z is an integer ranging from 9 to 110, preferably from 12 to 100.

7. A copolymer according to any one of claim 5 or 6, wherein
y+z is an integer ranging from 8 to 50, preferably from 9 to 45, preferably from 12 to 40, or
y+z is an integer ranging from 55 to 110, preferably from 60 to 100.

8. A copolymer according to any one of claims 5 to 7, wherein
y is a number ranging from 0 to 120, preferably 0 to 110, preferably from 5 to 100,
preferably from 9 to 95, preferably from 10 to 90, and
z is a number ranging from 0 to 120, preferably 0 to 110, preferably from 5 to 100,
preferably from 9 to 95, preferably from 10 to 90.

9. A copolymer according to any one of claims 5 to 8, wherein R^{y} and R^{z} are independently in D- or L- configuration, preferably R^{y} is the lateral chain of a hydrophobic L-amino acid, and R^{z} is the lateral chain of a hydrophobic D-amino acid, preferably R^{y} and R^{z} are independently chosen from the lateral chain of L-leucine, L-phenylalanine, L-tyrosine, γ-benzyl-L-glutamate, γ-tert-butyl-L-glutamate, L-cyclohexylglycine, D-leucine, D-phenylalanine, D-cyclohexylglycine, D-tyrosine, γ-benzyl-D-glutamate, γ-tert-butyl-D-glutamate.

10. A method of preparation of a copolymer according to any one of claims 1 to 9 by polymerization of sarcosine derivates and amino acid derivates, wherein derivates are represented by formulas III and IV as follow 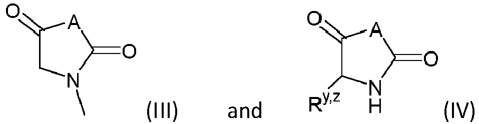 wherein
A is O or S,
R^{y}, R^{z} are as defined in any of claims 5 to 9.

11. Nanoparticles comprising copolymer according to any one of claims 1 to 9 or prepared according to the method of claim 10.

12. Nanoparticles according to claim 11 further comprising at least one active compound, preferably selected from hydrophobic compounds, active pharmaceutical ingredients.

13. Nanoparticles according to any one of claim 11 or 12, having
- a hydrodynamic diameter lower than 400 nm, preferably ranging from 5 to 200 nm, and/or
- a polydispersity index lower than 0.70, preferably ranging from 0.02 to 0.70; and/or
- a loading efficiency of more than 20%, preferably more than 30%.

14. A method of preparing nanoparticles according to any one of claims 11 to 13 comprising the following steps :
- preparing an organic solution containing the copolymer according to any one of claims 1 to 9,
- mixing the organic solution with an aqueous solution upon stirring,
- removing the organic solvent.

15. A method of preparing nanoparticles according to claim 14 wherein nanoparticles further comprise a hydrophobic compound, said method comprising the following steps:
- preparing an organic solution containing the copolymer according to any one of claims 1 to 9 and at least a hydrophobic compound,
- mixing the organic solution with an aqueous solution upon stirring,
- removing the organic solvent.
